# EUROPÄISCHE PATENTSCHRIFT

(11) **EP 1 046 371 B1**
(45) Veröffentlichungstag und Bekanntmachung des Hinweises auf die Patenterteilung: **17.03.2004**
(21) Anmeldenummer: 00107097.8
(22) Anmeldetag: 06.04.2000
(51) Int. Cl.: A61B 1/00, A61B 1/12

(54) **Endoskop mit einer Einrichtung zur Säuberung der Optik am distalen Ende**
Endoscope with cleaning device for the optics at its distal end
Endoscope avec une système de nettoyage de l'optique dans l'extremité distale

(30) Priorität: 19.04.1999 DE 19917624
(43) Veröffentlichungstag der Anmeldung: 25.10.2000
(73) Patentinhaber: Ferton Holding SA, 2800 Delémont (CH)
(72) Erfinder: Schmidt, Joachim, Dr., 51427 Bergisch-Gladbach (DE); Raabe, Thorsten, Dr., 88682 Salem-Buggensegel (DE)
(74) Vertreter: Gauger, Hans-Peter, Dipl.-Ing.

(56) Entgegenhaltungen:
- EP-A- 0 437 229
- US-A- 4 919 113

## Beschreibung

Die Erfindung bezieht sich auf ein Endoskop, welches an einem distalen Ende eines länglichen Hohlschaftes einen Lichtträger und eine Optik aufweist, wobei längs des Hohlschaftes eine Fluidleitung verlegt ist, über welche zur Reinigung einer stimseitigen Sichtfläche der Optik eine Spülflüssigkeit zugeleitet wird.

Aus der EP 0 497 347 A2 ist ein Laparoskop mit den vorerwähnten Merkmalen bekannt, bei welchem die Optik für die Betrachtung einer Körperhöhle während einer Laparoskopie benutzt wird. Mit der Optik ist eine Videokamera verbunden, welche das optische Bild der Körperhöhle auf den Bildschirm eines Farbmonitors überträgt. Damit das übertragene Videobild nicht nur für die Dauer einer Diagnose, sondern insbesondere auch für die Dauer eines operativen Eingriffes eine stets gleichbleibende Schärfe beibehält, ist die Fluidleitung an eine an dem distalen Ende des Hohlschaftes angeordnete Spritzdüse angeschlossen. An die Spritzdüse wird bsp. eine Salzlösung unter einem Druck von etwa 200 bis 350 mm Hg über die Fluidleitung zugeleitet, um gegen die Sichtfläche der Optik versprüht zu werden, sobald für die Optik eine Sichtbehinderung durch auftretende Verunreinigungen festgestellt werden muß. Die Zuleitung der Salzlösung oder auch einer anderen Reinigungs- oder Spülflüssigkeit an die Spritzdüse ist dabei mit der Betätigung eines Trompetenventils beeinflußbar, welches mit der Fluidleitung verbunden ist.

Für ein Arbeiten mit einem Endoskop, bei welchem eine Reinigung der Optik mit einer Spülflüssigkeit vorgesehen ist, die versprüht wird, sobald an der Optik eine Sichtbehinderung auftritt, kann es sich als nachteilig erweisen, daß bei einer Unterbrechung des Reinigungsprozesses meistens einzelne Flüssigkeitstropfen an der Sichtfläche der Optik verbleiben. Solche verbleibenden Flüssigkeitstropfen können zu einer kritischen Verzerrung des mit der Optik aufgenommenen und bsp. auf den Bildschirm eines Farbmonitors übertragenen Videobildes führen. Die Versprühung einer Spülflüssigkeit für eine Reinigung der Optik bei den angegebenen Drücken kann aber auch dazu führen, daß der während eines operativen Eingriffs mit einer Flüssigkeit oder mit einem Gas getrennt geregelte Druckhaushalt der betrachteten Körperhöhle zumindest zu dem Zeitpunkt übermäßig gestört wird, wo die für eine Reinigung der Optik gesteuerte Zuleitung der Spülflüssigkeit unterbrochen wird. In der unmittelbaren Nähe der Optik kommt es dabei dann zu einer kritischen Druckveränderung, welche die Körperflüssigkeit an die Optik heranführen läßt und so zu einer Trübung des übertragenen Bildes führen kann.

Aus der DE 39 33 085 A1 ist für die Anwendung bei einem Koloskop eine Reinigungseinrichtung bekannt, bei welcher mittels einer pulsierend arbeitenden Pumpe ein intermittierender Flüssigkeitsstrahl erzeugt wird, mit welchem eine Reinigung der Darmschleimhäute bsp. bei Darmspiegelungen bezweckt wird. Die eigentliche Untersuchung des Darmes soll dadurch keine Beeinträchtigung durch Verunreinigungen der Schleimhäute erfahren. Mit dem Flüssigkeitsstrahl am distalen Ende eines Hohlschaftes des Koloskops soll daneben auch eine Reinigung einer Optik des Koloskops durchgeführt werden, wobei aber dazu keine näheren Erläuterungen angegeben sind. Festgehalten ist nur, daß mit einem intermittierend arbeitenden Flüssigkeitsstrahl, der durch Betätigung eines mit der pulsierend arbeitenden Pumpe verbundenen Fußschalters beeinflußbar ist, hauptsächlich eine optimale Reinigung der Darmschleimhäute zu erhalten ist.

Der Erfindung liegt die Aufgabe zugrunde, bei einem Endoskop der eingangs genannten Art Vorkehrungen zu treffen, die eine an der Optik auftretende Sichtbehinderung auch dann verhindern oder zumindest weitgehend reduzieren läßt, wenn die Reinigung unterbrochen wird.

Diese Aufgabe wird erfindungsgemäß gelöst mit einem Endoskop in der Ausbildung gemäß dem Patentanspruch 1. Mit den Merkmalen der weiteren Patentansprüche werden entsprechend vorteilhafte und zweckmäßige Weiterbildungen des erfindungsgemäßen Endoskops erhalten.

Durch die mit dem erfindungsgemäßen Endoskop intervallmäßig und abwechselnd derart geregelte Zuleitung der Spülflüssigkeit, daß anfänglich ein relativ starker Flüssigkeitsstrahl gegen die stirnseitige Sichtfläche der Optik ausgerichtet wird und dann die Zuleitung der Spülflüssigkeit kurz vor einer Unterbrechung dieses relativ starken Flüssigkeitsstrahls auf eine Fließrate reduziert wird, welche an der Sichtfläche der Optik die Ausbildung eines derart minimalen Flüssigkeitsfilms ergibt, daß damit eine Sichtbehinderung vermieden wird, läßt sich auf überraschend einfache Weise eine sonst störende Ansammlung von Flüssigkeitstropfen oder eines verbleibenden Sprühnebels an der Sichtfläche der Optik vermeiden. Die Schaffung eines solchen minimalen Flüssigkeitsfilms ergibt auch die Möglichkeit, daß der eigentliche Reinigungsprozeß der Optik mit einem Flüssigkeitsstrahl durchgeführt wird, der auch wesentlich stärker sein kann als es für eine Beseitigung von Verunreinigungen unbedingt erforderlich ist. Die fallweise erforderliche Reinigung der Optik kann daher auch in einer kürzeren Zeitdauer durchgeführt werden als es im Vergleich mit einem weniger starken Flüssigkeitsstrahl möglich wäre.

Die Ausbildung des minimalen Flüssigkeitsfilms an der Sichtfläche der Optik kann entweder nur für eine kurze Zeitdauer vorgesehen werden, bevor dann anschließend die Zuleitung der Spülflüssigkeit ausgeschaltet wird. Es kann alternativ aber auch vorgesehen werden, diese Ausbildung eines minimalen Flüssigkeitsfilms bis zu einem Zeitpunkt stetig zu unterhalten, in welchem der Reinigungsprozeß der Optik wieder mit einem relativ starken Flüssigkeitsstrahl aufgenommen wird. Beide Möglichkeiten sind bsp. mit der Eingliederung einer Regeleinrichtung durchführbar, mit welcher die Fließrate der Spülflüssigkeit auf die jeweils passenden Werte eingestellt werden kann. Die Regeleinrichtung kann so bsp. mit einem einfachen Regelventil ausgeführt sein, welches die Zuleitung der Spülflüssigkeit über die Fluidleitung mit zwei definierten Regelstufen steuert, in welchen eine größere Flüssigkeitsmenge pro Zeiteinheit für den relativ starken Flüssigkeitsstrahl in der einen Stufe und eine weitgehend reduzierte Flüssigkeitsmenge pro Zeiteinheit für die Schaffung des minimalen Flüssigkeitsfilmes in der zweiten Stufe an die stirnseitige Sichtfläche der Optik ausgeliefert wird.

Ein Ausführungsbeispiel des erfindungsgemäßen Endoskops ist in der Zeichnung schematisch dargestellt und wird nachfolgend näher erläutert. Es zeigt
- Fig. 1: eine Schemadarstellung eines Endoskops mit einer integrierten Einrichtung zur Reinigung einer Optik am distalen Ende eines Hohlschaftes des Endoskops und
- Fig. 2: die Einzelheit in einem größeren Maßstab, welche für die Reinigung der Optik am distalen Ende des Hohlschaftes des Endoskops maßgebend ist.

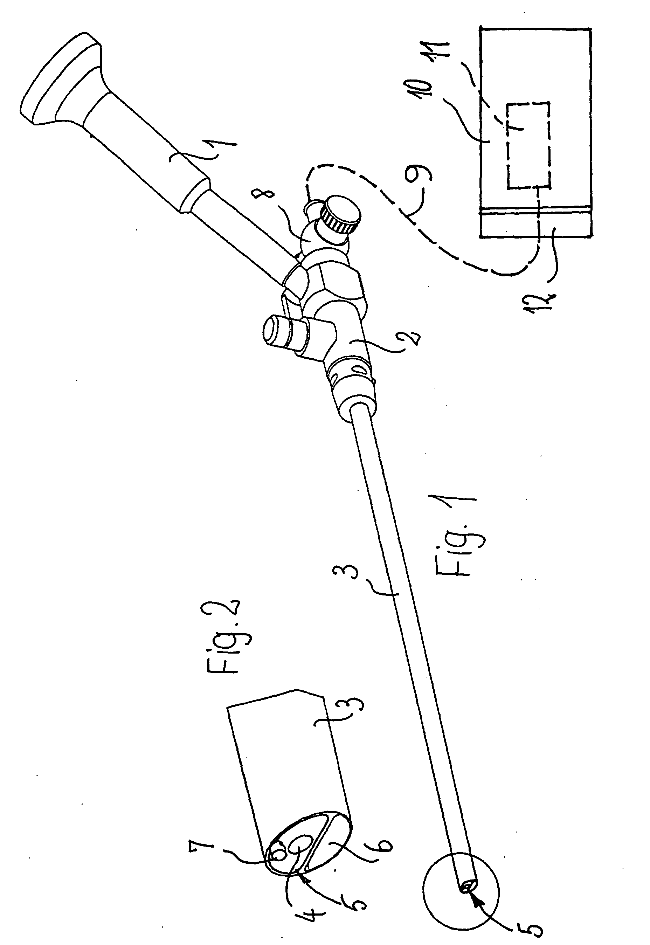

Das in Fig. 1 schematisch dargestellte Endoskop weist ein abgewinkeltes Okular 1 auf, das von einem Grundkörper 2 absteht. Von dem Grundkörper 2 erstreckt sich ein länglicher Hohlschaft 3 nach vorne, der zur Aufnahme eines Lichtträgers und einer Optik dient, die beide für eine diagnostische Betrachtung von Körperhöhlen und Hohlorganen dienen und am distalen Ende des Hohlschaftes angeordnet sind. Der Hohlschaft 3 des Endoskops kann daneben auch zur Aufnahme von Instrumenten ausgebildet sein, die einen minimalinvasiven operativen Eingriff erlauben. Der Hohlschaft 3 wäre dafür noch mit einem Schlauchanschluß verbunden, um ein Ausspülen einer Körperhöhle bzw. eines Hohlorgans zu ermöglichen.

Die Optik des Endoskops ist mit einer Linse 4 am distalen Ende des Hohlschaftes 3 ausgebildet. Sie erfaßt das Bild einer diagnostisch betrachteten Körperhöhle zur Übertragung an das Okular 1 und ggf. auf den Bildschirm eines Farbmonitors durch eine mit dem Okular verbundene Videokamera. Mit der Linse 4 ist eine stimseitige Sichtfläche 5 der Optik ausgebildet, wobei in dieser Sichtfläche auch das Austrittsende eines Lichtträgers angeordnet ist unmittelbar neben dem Austrittsende eines Arbeitskanals 6, in welchem bsp. ein für einen minimalinvasiven operativen Eingriff benutztes Instrument aufgenommen sein kann. In diesem Arbeitskanal kann dann auch die zusätzliche Leitung aufgenommen sein, über welche die Flüssigkeit zugeleitet wird, die für ein Ausspülen einer Körperhöhle benutzt wird. Alternativ kann über diese zusätzliche Leitung auch ein Gas zugeleitet werden, mit welchem der Druckhaushalt der Körperhöhle gesteuert wird.

Neben der Linse 4 ist das vorstehende Austrittsende 7 einer Fluidleitung angeordnet, die in dem Hohlschaft 3 verlegt ist. Die Fluidleitung ist an dem Grundkörper 2 des Endoskops mit einem Regelventil 8 verbunden, welches die herkömmliche Ausbildung eines Trompetenventils aufweisen kann und über eine Verbindungsleitung 9 bsp. mit einem Flüssigkeitsbehälter 10 verbunden ist, der eine für einen Reinigungsprozeß der Optik benötigte Spülflüssigkeit enthält. Die Spülflüssigkeit kann durch eine in diesem Behälter angeordnete Pumpe 11 in die Fluidleitung zugeleitet werden. In der Anschlußleitung 9 an das Regelventil 8 kann noch eine besondere Regeleinrichtung 12 eingebaut sein, um damit für ein optimale Ausgestaltung des Reinigungsprozesses eine Feineinstellung von unterschiedlichen Fließraten der Spülflüssigkeit zu erhalten.

Bei einem Arbeiten mit dem vorbeschriebenen Endoskop wird ein Reinigungsprozeß der Optik immer dann veranlaßt, wenn eine Sichtbehinderung der Optik als Folge von Verunreinigungen über ein entsprechend undeutliches Bild festgestellt werden muß. Durch eine Betätigung primär des Regelventils 8 an dem Grundkörper 2 des Endoskops wird dafür dann die Zuleitung der Spülflüssigkeit an das Austrittsende 7 der Fluidleitung derart gesteuert, daß gegen die stirnseitige Sichtfläche 5 der Optik anfänglich ein relativ starker Flüssigkeitsstrahl ausgerichtet wird. Die Zuleitung der Spülflüssigkeit mit einem solchen anfänglich starken Fluidstrahl wird dann nach einer wählbaren Zeitdauer, die sich nach der Effizienz des Fluidstrahls bei diesem Reinigungsprozeß richtet, auf eine Fließrate reduziert, die an der Sichtfläche 5 der Optik die Ausbildung eines derart minimalen Flüssigkeitsfilms ergibt, daß damit eine Sichtbehinderung vermieden wird. Die Ausbildung eines solchen minimalen Flüssigkeitsfilms wird mit einer angepaßt zweistufigen Ausbildung des Regelventils 8 erreicht, wobei in der einen Stufe der relativ starke Flüssigkeitsstrahl mit einer entsprechend großen Durchflußmenge der Spülflüssigkeit erhalten wird, während mit einer angepaßt reduzierten Durchflußmenge die Ausbildung des minimalen Flüssigkeitsfilms dann in der zweiten Regelstufe des Regelventils erreicht wird.

Die Ausbildung des minimalen Flüssigkeitsfilms an der stirnseitigen Sichtfläche 5 der Optik kann entweder nur momentan vorgenommen werden, um dann unmittelbar anschließend die Zuleitung der Spülflüssigkeit an das Austrittsende 7 der Fluidleitung vollständig auszuschalten. Für diesen Zweck kann auch von der Schwerkraft der Spülflüssigkeit in der Weise Gebrauch gemacht werden, daß mit einer von oben nach unten ausgerichteten Fließrichtung einer Restmenge der Spülflüssigkeit eine flächenabdeckende Verteilung über die Stirnfläche der Optik erhalten wird.

Alternativ kann auch vorgesehen werden, daß eine für die Sichtfläche der Optik gewünschte Ausbildung eines minimalen Flüssigkeitsfilms nach der Beendigung des Reinigungsprozesses mit einer stetig fortgesetzten Zuleitung der Spülflüssigkeit solange beibehalten wird, bis wieder die Notwendigkeit für eine Wiederholung des Reinigungsprozesses erkannt und dafür dann wieder auf den relativ starken Flüssigkeitsstrahl übergewechselt wird.

Um eine optimale Ausbildung des minimalen Flüssigkeitsfilms zu erhalten, sollte zweckmäßig eine Spülflüssigkeit verwendet werden, welche die Sichtfläche der Optik mit einer vorbestimmten minimalen Schichtdicke benetzen läßt. Die Erreichbarkeit einer solchen minimalen vorbestimmten Schichtdicke kann dabei weiter unterstützt werden durch das Vorsehen einer Oberflächenstruktur für die Sichtfläche der Optik, welche eine entsprechend angepaßte Benetzung mit der Spülflüssigkeit erwarten läßt. Weitere Einflußgrößen können mit einer angepaßten Regelung der Fließrate für die Spülflüssigkeit angedacht werden, wenn dafür die Regeleinrichtung 12 entsprechend feinstufig ausgeführt wird, sowie auch mit einer angepaßten Anordnung und Ausbildung des Austrittsendes der Fluidleitung. Es kann dafür bsp. eine schlitzförmige Einfach- oder auch Mehrfachausbildung der Austrittsöffnung für die Spülflüssigkeit angedacht werden.

Die Ausbildung eines minimalen Flüssigkeitsfilms an der Sichtfläche der Optik kann in aller Regel für völlig ausreichend angesehen werden, um während des Arbeitens mit dem Endoskop eine ständig gleichbleibende Qualität und Schärfe des mit der Optik übertragenen Bildes in Verbindung mit einem Reinigungsprozeß der Optik zu gewährleisten. Für eine optimale Beseitigung von Verschmutzungen der Optik kann auch noch die ergänzende Maßnahme realisiert werden, im Bedarfsfall auch noch einen Luftstrahl gegen die Sichtfläche der Optik auszurichten. Es wäre dafür nur eine weitere separate Leitung in dem Hohlschaft des Endoskops zu verlegen, um über über diese Leitung dann einen solchen zusätzlichen Luftstrahl gegen die Sichtfläche der Optik ausrichten zu können.

## Patentansprüche

1. Endoskop, welches einen länglichen Hohlschaft (3) zur Aufnahme eines Lichtträgers und einer Optik (4) sowie einer längs des Hohlschaftes (3) verlegten Fluidleitung aufweist, über welche eine Spülflüssigkeit an das distale Ende des Hohlschaftes zur Reinigung einer stirnseitigen Sichtfläche (5) der Optik (4) zugeleitet wird,
**dadurch gekennzeichnet, daß** das Endoskop Mittel aufweist um die Zuleitung der Spülflüssigkeit intervallmäßig und abwechselnd derart zu regeln, daß anfänglich ein relativ starker Flüssigkeitsstrahl gegen die stirnseitige Sichtfläche (5) der Optik (4) ausgerichtet wird und die Zuleitung der Spülflüssigkeit kurz vor einer Unterbrechung dieses relativ starken Flüssigkeitsstrahls auf eine Fließrate reduziert wird, welche an der Sichtfläche (5) der Optik (4) die Ausbildung eines derart minimalen Flüssigkeitsfilms ergibt, daß damit eine Sichtbehinderung an der Optik vermieden wird.

2. Endoskop nach Anspruch 1,
**dadurch gekennzeichnet, daß** die Zuleitung der Spülflüssigkeit nach der Ausbildung des minimalen Flüssigkeitsfilms bis zu einer Wiederaufnahme einer mit dem relativ starken Flüssigkeitsstrahl wiederholten Reinigung der Sichtfläche (5) der Optik (4) ausgeschaltet wird.

3. Endoskop nach Anspruch 1,
**dadurch gekennzeichnet, daß** die Ausbildung des minimalen Flüssigkeitsfilms stetig beibehalten wird bis zu einer Wiederaufnahme einer mit dem relativ starken Flüssigkeitsstrahl wiederholten Reinigung der Sichtfläche (5) der Optik (4).

4. Endoskop nach einem der Ansprüche 1 bis 3,
**dadurch gekennzeichnet, daß** die Ausbildung des minimalen Flüssigkeitsfilms an der Sichtfläche (5) der Optik (4) mit einer Unterstützung durch die Schwerkraft der Spülflüssigkeit durch die Vorgabe einer Fließrichtung für die Spülflüssigkeit von oben nach unten durchgeführt wird.

5. Endoskop nach einem der Ansprüche 1 bis 4,
**dadurch gekennzeichnet, daß** eine Spülflüssigkeit verwendet wird, die bei der Ausbildung des minimalen Flüssigkeitsfilms für eine Benetzung der Sichtfläche (5) der Optik (4) mit einer vorbestimmten minimalen Schichtdicke geeignet ist.

6. Endoskop nach Anspruch 5,
**dadurch gekennzeichnet, daß** die Sichtfläche (5) der Optik (4) mit einer Oberflächenstruktur versehen ist, die sich für eine Benetzung mit einer vorbestimmten minimalen Schichtdicke der Spülflüssigkeit eignet.

7. Endoskop nach einem der Ansprüche 1 bis 6,
**dadurch gekennzeichnet, daß** mit der Fluidleitung eine Regeleinrichtung (12) verbunden ist, welche die Spülflüssigkeit auf unterschiedliche Fließraten des gegen die Sichtfläche (5) der Optik (4) ausgerichteten Flüssigkeitsstrahls einstellen läßt.

8. Endoskop nach Anspruch 7,
**dadurch gekennzeichnet, daß** die Regeleinrichtung (12) mit einem Regelventil. (8) ausgebildet ist, das sich auf wenigstens zwei definierte Regelstufen einstellen läßt, in denen die Spülflüssigkeit mit einer maximalen und mit einer minimalen Durchflußmenge an die Sichtfläche der Optik zugeleitet wird.

9. Endoskop nach einem der Ansprüche 1 bis 8,
**dadurch gekennzeichnet, daß** die Fluidleitung eine schlitzförmig ausgebildete Austrittsöffnung aufweist, deren Schlitzgröße zur Erzielung einer gleichmäßigen Ausbreitung der Spülflüssigkeit über die Sichtfläche (5) der Optik (4) bemessen ist.

10. Endoskop nach einem der Ansprüche 1 bis 9,
**dadurch gekennzeichnet, daß** die Fließrate der Spülflüssigkeit derart geregelt wird, daß der minimale Flüssigkeitsfilm an der Sichtfläche (5) der Optik (4) erhalten bleibt, wenn die Zuleitung der Spülflüssigkeit unterbrochen ist.

11. Endoskop nach einem der Ansprüche 1 bis 10,
**dadurch gekennzeichnet, daß** längs des Hohlschaftes (3) eine separate Leitung für die Zuleitung eines Luftstrahles verlegt ist, der an der Sichtfläche (5) der Optik (4) die Ausbildung des minimalen Flüssigkeitsfilms unterstützt.

12. Endoskop nach Anspruch 11,
**dadurch gekennzeichnet, daß** die separate Leitung für die Zuleitung eines Luftstrahls außerhalb des Hohlschaftes (3) verlegt und durch Klammern od. dgl. an dem Hohlschaft gehalten ist.

## Claims

1. Endoscope, comprising an elongated hollow shaft (3) for receiving a light carrier and optics (4) as well as a fluid line extending along the hollow shaft (3) for passing a purging fluid to the distal end of the hollow shaft for cleaning a front vision face (5) of the optics (4),
**characterised in that** the endoscope comprises means for distributing the purging fluid in intervals and alternately in such a manner that initially a relatively strong fluid jet will be directed against the front vision face (5) of the optics (4) and that the distribution of the purging fluid will be reduced shortly before an interruption of this relatively strong fluid jet to a flow rate which results in the formation of such a minimal fluid film on the vision face (5) of the optics (4) that thereby any impediment of visibility will be avoided at the optics.

2. Endoscope according to claim 1,
**characterised in that** the distribution of the purging fluid is switched-off after the formation of the minimal fluid film until the cleaning of the vision face (5) of the optics (4) will again be taken up with a relatively strong fluid jet.

3. Endoscope according to claim 1,
**characterised in that** the formation of the minimal fluid film is continuously maintained until the cleaning of the vision face (5) of the optics (4) will be repeated with a relatively strong fluid jet.

4. Endoscope according to any of the claims 1 to 3,
**characterised in that** the formation of the minimal fluid film on the vision face (5) of the optics (4) is carried out with a support by the gravity of the purging fluid by allowing a flow direction of the purging fluid in a downward direction from above.

5. Endoscope according to any of the claims 1 to 4,
**characterised in that** a purging fluid is being used which for the formation of the minimal fluid film will be adapted for wetting the vision face (5) of the optics (4) with a predetermined minimal layer thickness.

6. Endoscope according to claim 5,
**characterised in that** the vision face (5) of the optics (4) is provided with a surface structure that allows a wetting with a predetermined minimal layer thickness of the purging fluid.

7. Endoscope according to any of the claims 1 to 6,
**characterised in that** a control means (12) is connected with the fluid line which is adapted to adjust the purging fluid to different flow rates of its fluid jet which is directed against the vision face (5) of the optics (4).

8. Endoscope according to claim 7,
**characterised in that** the control means (12) comprises a control valve (8) which may be adjusted to at least two defined control ranges in which the purging fluid is distributed to the vision face (5) of the optics (4) with a maximum and with a minimum flow quantity.

9. Endoscope according to any of the claims 1 to 8,
**characterised in that** the fluid line comprises a slotted exit opening the slot size of which is dimensioned such as to allow a uniform distribution of the purging fluid across the vision face (5) of the optics (4).

10. Endoscope according to any of the claims 1 to 9,
**characterised in that** the flow range of the purging fluid is controlled such that the minimal fluid film will remain on the vision face (5) of the optics (4) when the distribution of the purging fluid will be interrupted.

11. Endoscope according to any of the claims 1 to 10,
**characterised in that** a separate line for the distribution of an air jet is passed along the hollow shaft (3) which air jet will support the formation of the minimal fluid film on the vision face (5) of the optics (4).

12. Endoscope according to claim 11,
**characterised in that** the separate line for the distribution of an air jet is passed on the exterior of the hollow shaft (3) and is fixed to the hollow shaft by means of clamps or the like.

## Revendications

1. Endoscope comprenant une tige creuse allongée (3) à recevoir un porteur de lumière et un système optique (4) ainsi qu'une canalisation de fluide placée le long de ladite tige creuse (3), via laquelle un liquide d'irrigation est introduit vers l'extrémité distale de ladite tige creuse pour le nettoyage d'une aire de visée (5) du côté de la face dudit système optique,
**caractérisé en ce que** l'endoscope comprend des moyens à régler l'alimentation dudit fluide d'irrigation en intervalles et de manière alternative d'une telle façon, que d'abord un jet relativement fort du liquide soit orienté vers ladite aire de visée (5) du côté de la face du système optique (4) et que, brièvement avant une interruption de ce jet de liquide relativement fort, l'alimentation du fluide d'irrigation soit réduit à un taux de fluage, qui foumit le développement d'un film de liquide à ladite aire de visée (5) du système optique (4), qui est minime afin d'éviter ainsi une gêne de vue au système optique.

2. Endoscope selon la revendication 1,
**caractérisé en ce que** l'alimentation du fluide d'irrigation est coupée après le développement dudit film de liquide minime jusqu'à une reprise d'un nettoyage de ladite aire de visée (5) du système optique (4), qui est répété moyennant le jet de liquide relativement fort.

3. Endoscope selon la revendication 1,
**caractérisé en ce que** le développement d'un film de liquide minime est constamment maintenu jusqu'à une reprise du nettoyage de ladite aire de visée (5) du système optique (4), qui est répété moyennant le jet de liquide relativement fort.

4. Endoscope selon une quelconque des revendications 1 à 3,
**caractérisé en ce que** le développement dudit film de liquide minime à ladite aire de visée (5) du système optique (4) se fait par la détermination d'un sens d'écoulement du liquide d'irrigation de l'haut en bas, à l'assistance par la force de gravité du liquide d'irrigation.

5. Endoscope selon une quelconque des revendications 1 à 4,
**caractérisé en ce qu'**un liquide d'irrigation est utilisé, qui est approprié à mouiller ladite aire de visée (5) du système optique (4) à une épaisseur de couche minimale prédéterminée, au développement dudit film de liquide minimal.

6. Endoscope selon la revendication 5,
**caractérisé en ce que** ladite aire de visée (5) dudit système optique (4) est munie d'une structure superficielle, qui est appropriée au mouillage à une épaisseur de couche minimale prédéterminée du liquide d'irrigation.

7. Endoscope selon une quelconque des revendications 1 à 6,
**caractérisé en ce qu'**un moyen de réglage (12) est relié à ladite canalisation de fluide, qui permet l'ajustement du liquide d'irrigation aux taux de fluages différents du jet de liquide orienté vers ladite aire de visée (5) du système optique (4).

8. Endoscope selon la revendication 7,
**caractérisé en ce que** ledit moyen de réglage (12) est configuré à une vanne-pilote (8) ajustable à au moins deux niveaux définis de réglage, auxquels le liquide d'irrigation est alimenté à ladite aire de visée du système optique à un débit de passage maximal et un débit de passage minimal.

9. Endoscope selon une quelconque des revendications 1 à 8,
**caractérisé en ce que** ladite canalisation de fluide comprend un orifice de décharge à configuration en fente, dont la grandeur de la fente est dimensionnée pour l'achèvement d'une diffusion homogène du liquide d'irrigation sur ladite aire de visée (5) du système optique.

10. Endoscope selon quelconque des revendications 1 à 9,
**caractérisé en ce que** le taux de fluage du liquide d'irrigation est réglé d'une telle manière, que le film de liquide minimal soit maintenu à ladite aire de visée (5) du système optique (4), quand l'alimentation du liquide d'irrigation est coupée.

11. Endoscope selon une quelconque des revendications 1 à 10,
**caractérisé en ce qu'**une canalisation séparée pour l'alimentation d'un jet d'air est placée le long de ladite tige creuse (3), ce jet d'air assistant le développement du film de liquide minimal.

12. Endoscope selon la revendication 11,
**caractérisé en ce que** ladite canalisation séparée pour l'alimentation d'un jet d'air est placée à l'extérieur de ladite tige creuse (3) et est fixée moyennant des pinces ou éléments similaires.
